(19) Europäisches Patentamt
European Patent Office
Office européen des brevets

(11) Numéro de publication: **0 098 783**
**B1**

(12) # FASCICULE DE BREVET EUROPEEN

(45) Date de publication du fascicule du brevet: **21.08.85**

(51) Int. Cl.⁴: **C 07 C 85/04**, C 07 C 87/60

(21) Numéro de dépôt: **83420103.0**

(22) Date de dépôt: **21.06.83**

(54) **Procédé de préparation de halogéno anilines.**

(30) Priorité: **29.06.82 FR 8211617**

(43) Date de publication de la demande:
**18.01.84 Bulletin 84/3**

(45) Mention de la délivrance du brevet:
**21.08.85 Bulletin 85/34**

(84) Etats contractants désignés:
**AT BE CH DE FR GB IT LI LU NL SE**

(56) Documents cités:
**FR - A - 2 389 596**
**US - A - 4 117 013**

**CHEMICAL ABSTRACTS, vol. 94, no. 9, 2 mars 1981, page 646, no. 64770z, Columbus, Ohio, USA S.A. KONDRATOV et al.: "Catalysis by copper complexes in the reaction of o-nitrochlorobenzene with ammonia"**

(73) Titulaire: **RHONE-POULENC AGROCHIMIE, 14-20, rue Pierre Baizet, F-69009 Lyon (FR)**

(72) Inventeur: **Ratton, Serge, Vaulx-Milieu, F-38290 La Verpillière (FR)**

(74) Mandataire: **Chrétien, François et al, RHONE-POULENC AGROCHIMIE BP 9163, F-69263 Lyon Cedex 09 (FR)**

**EP 0 098 783 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation d'anilines éventuellement halogénées, par ammonolyse d'un (poly) halogénobenzène. Ces anilines et notamment la dichloro-3,5 aniline sont des intermédiaires pour la fabrication de matières actives agrochimiques.

Il est connu de préparer des dihalogéno-3,5 anilines par réaction d'ammoniac gazeux, liquide ou en solution aqueuse, en présence d'un catalyseur au cuivre, celui-ci pouvant être associé à un complexant. Ce type de réaction est peu productif dans des conditions économiques la réaction étant lente, d'une sélectivité insuffisante et de rendement limité, notamment par ammolyse ultérieure du bon produit obtenu. Ceci est particulièrement net dans le cas de la dichloro-3,5 aniline, dans la préparation de laquelle on obtient, par ce procédé, une importante proportion de diamino-1,3 chlor-5 benzène. Ces inconvénients empêchent donc l'utilisation de cette voie à l'échelle industrielle dans de bonnes conditions. Or, les produits issus de cette réaction et notamment la dichloro-3,5 aniline sont des intermédiaires de matières actives agrochimiques dont la demande croissante entraîne celle de ces intermédiaires.

La présente invention a trait à un nouveau procédé du type ci-dessus mais ne présentant pas les inconvénients précités.

Plus précisément l'invention concerne un procédé de préparation d'anilines éventuellement halogénées, de formule générale:

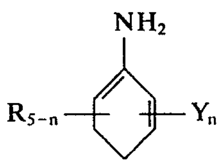

dans laquelle:

— Y représente un ou des atomes d'halogènes, identiques ou différents,
— R représente un ou plusieurs substituants choisis dans le groupe comprenant un atome d'hydrogène et un radical alcoyle contenant de 1 à 4 atomes de carbone ou alcoxyle contenant de 1 à 4 atomes de carbone et
— n est un nombre entier de 0 à 5, par ammonolyse d'halogénobenzènes de formule:

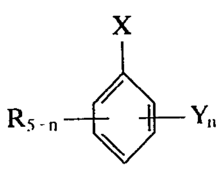

dans laquelle:

— Y et R ont les mêmes significations que précédemment et
— X représente un atome d'halogène, avec une solution aqueuse d'ammoniac, en présence de cuivre comme catalyseur et d'un complexant du cuivre, caractérisé en ce qu'on utilise comme complexant un dérivé de l'hydroxy-8 quinoléine.

Les composés halogénobenzènes de départ définis ci-dessus comprennent notamment les monohalogénobenzènes jusqu'aux hexahalogénobenzènes et en particulier les trihalogénobenzènes et tout particulièrement les tri 1,3,5 halogénobenzènes. Les atomes d'halogène de ces composés sont notamment le brome, l'iode et de préférence le chlore. Par ailleurs, le substituant R est de nature à ne pas modifier sensiblement le processus d'ammonolyse, de préférence un atome d'hydrogène et/ou un radical méthyle et/ou méthoxy. Enfin, n a de préférence une valeur entière de 0 à 3.

L'ammonolyse selon l'invention est effectuée à l'aide d'ammoniac en solution aqueuse, de préférence concentrée pour travailler dans des conditions économiques. Cette présentation n'est pas limitative et toute autre composition aqueuse libérant de l'ammoniac dans des conditions équivalentes peut être utilisée. La quantité d'ammoniac à apporter peut être stoechiométrique, inférieure à la stoechiométrie mais de préférence supérieure, c'est-à-dire correspondant à un rapport molaire, par rapport au produit de départ, de 1/1 à 20/1 et de préférence de 3/1 à 10/1.

Le catalyseur au cuivre peut se présenter sous forme de cuivre métal ou d'oxydes ou d'hydroxyes ou de sels cuivreux ou cuivriques d'acides minéraux ou organiques, en particulier halogénures et de préférence chlorures, acétate, oxyde ou cyanure. La préférence est donnée aux composés cuivreux. Le catalyseur est utilisé à raison de, exprimé en cuivre, 0,08 à 30% et de préférence de 1 à 10% en moles

par rapport au produit de départ. Ce catalyseur peut être engagé sur un support inerte mais est utilisé de préférence tel quel dans le milieu. En effet, à la température de réaction, il se solubilise, ce qui permet de travailler en catalyse homogène.

Le complexant particulier selon l'invention est un dérivé de l'hydroxy-8 quinoléine. Par dérivé de l'hydroxy-8 quinoléine on entend l'hydroxy-8 quinoléine proprement dite ainsi que ses dérivés, notamment substitués sur le noyau, comme par exemple l'acide hydroxy-8 quinoléine sulfonique-5. L'addition de ces composés améliore considérablement le rendement de la réaction, ce qui est d'autant plus surprenant que des complexants très voisins, comme la quinoléine ou l'hydroxy-2 quinoléine, ne sont guère plus efficaces que le cuivre seul. Le complexant particulier selon l'invention est utilisé de manière que le rapport molaire complexant/cuivre est compris entre 0,1/1 et 5/1 et de préférence entre 0,5/1 et 2/1.

Avantageusement, on peut améliorer la sélectivité de la réaction par l'addition, au milieu réactionnel, d'un solvant de l'halogénobenzène, miscible à l'eau et dépourvu d'effet désactivant du cuivre. Ce solvant est utilisé généralement en quantité telle que le rapport pondéral solvant/halogénobenzène de départ est compris entre 0,1/1 et 10/1 et de préférence entre 0,5/1 et 4/1. Ce solvant peut être de matière chimique variée. On a en particulier obtenu des résultats intéressants avec de préférence la N-méthyl pyrolidone et la tétraméthylurée mais aussi avec la pyridine, l'éthanol, des polyéthylèneglycols de poids moléculaire allant de 400 à 1500 et le diglyme.

Le procédé selon l'invention s'effectue à température relativement élevée, généralement comprise entre 150 et 250° C et de préférence entre 170 et 220° C.

La pression totale, le plus souvent autogène, est généralement comprise entre 10 et 70 bars et de préférence entre 20 et 50 bars.

Le procédé selon l'invention peut être mis en œuvre d'une manière contiune ou discontinue. En raison des taux de conversion partiels, on opère de préférence en continu avec recyclage du ou des halogénobenzènes non transformés.

L'aniline finale est séparée du mélange de manière habituelle, par exemple en filtrant à chaud le chlorure d'ammonium formé, en vaporisant, par détente, l'ammoniac et l'eau que l'on peut recycler, puis en reprenant le phase organique résiduelle, soit par un solvant sélectif de l'halogénobenzène, soit par distillation. On obtient ainsi l'(halogéno) aniline dans la pureté désirée.

Les exemples suivants sont donnés à titre indicatif pour illustrer l'invention:

## Exemple 1

On charge dans un autoclave à agitation 0,5 g de trichloro-1,3,5 benzène, 2,9 g d'une solution aqueuse à 28% en poids d'ammoniac, 0,014 g (soit $1,5 \times 10^{-4}$ mole) de chlorure cuivreux ainsi que (sauf pour un essai) $1,5 \times 10^{-4}$ mole d'un complexant dont la nature varie, respectivement l'hydroxy-8 quinoléine et l'acide hydroxy-8 quinoléine sulfonique-5, comme exemples de complexant selon l'invention, et de thiourée et de quinoléine comme exemples de complexants connus. On chauffe le mélange réactionnel à 200° C et on maintient à cette température pendant 1 h 20 minutes. Puis on laisse refroidir le mélange, on ramène la pression à la pression atmosphérique, on dilue avec 10 ml d'eau et on extrait avec deux fois 13 ml d'éther isopropylique. On soumet la solution résultante à l'analyse par chromatographie en phase vapeur.

Le tableau suivant rassemble pour chaque complexant utilisé, le taux de transformation (TT %) du trichloro,1,3,5 benzène:

| Nature du complexant | TT % |
|---|---|
| Hydroxy-8 quinoléine | 39,4 |
| Acide hydroxy-8 quinoléine sulfonique-5 | 53 |
| sans (cuivre seul) | 12,3 |
| quinoléine | 16,3 |
| thiourée | 1,15 |

Ce tableau montre clairement respectivement l'augmentation de plus de 3 et de 4 fois du taux de transformation avec un complexant selon l'invention, par rapport au cuivre seul ou en présence d'un complexant connu.

Exemple 2

On opère comme à l'exemple 1 si ce n'est l'on utilise le mélange suivant:

| | |
|---|---|
| Trichloro-1,3,5 benzène | 1 g ($5,5 \times 10^{-3}$ mole) |
| Ammoniaque à 28% | 2,9 g ($23 \times 10^{-3}$ mole) |
| Chlorure cuivreux | 0,03 g ($0,3 \times 10^{-3}$ mole) |
| Hydroxy-8 quinoléine | 0,042 g ($0,3 \times 10^{-3}$ mole) |
| Solvant | 1 g |

en faisant varier la nature du solvant et en effectucant des essais respectivement à 170, 200 et 220° C.

Le tableau suivant rassemble, pour chaque solvant utilisé, le taux de transformation du trichloro-1,3,5 benzène ainsi que le rendement molaire en dichloro-3,5 aniline rapporté au trichloro-1,3,5 benzène transformé:

| Nature du solvant | Température °C | TT % | RT % |
|---|---|---|---|
| Tétraméthyluréee | 170 | 13,6 | 70,7 |
| | 200 | 51,0 | 57,1 |
| | 220 | 69,0 | 45,5 |
| Polyéthylène glycol de poids moléculaire 400 | 170 | 17,7 | 61,5 |
| | 200 | 47,3 | 48 |
| | 220 | 68 | 38,7 |
| Pyridine | 200 | 40 | 62,2 |

Exemple 3

On opère comme à l'exemple 1 si ce n'est que l'on utilise le mélange suivant:

| | |
|---|---|
| Trichloro-1,3,5 benzène | 1,0 g ($5,5 \times 10^{-3}$ mole) |
| Ammoniaque à 28% | 2,9 g ($23 \times 10^{-3}$ mole) |
| Chlorure cuivreux | 0,03 g ($0,3 \times 10^{-3}$ mole) |
| Hydroxy-8 quinoléine | 0,084 g ($0,6 \times 10^{-3}$ mole) |
| éthanol | 2,0 g |

et que la réaction est effectuée à 200° C.

Dans ces conditions, on obtient un taux de transformation de 44% du trichloro-1,3,5 benzène et un rendement en dichlor-3,5 aniline de 70% par rapport au trichloro-1,3,5 benzène transformé.

Exemple 4

On opère comme à l'exemple 2, à 200° C pendant 1 h 20 en remplaçant, mole à mole, l'hydroxy-8 quinoléine par l'acide hydroxy-8 quinoléine sulfonique-5 et un utilisant la N-méthylpyrrolidone-5.

Dans ces conditions, on obtient un taux de transformation de 71,5% du trichloro-1,3,5 benzène et un rendement de 60% en dichloro-3,5 aniline sur le trichloro-1,3,5 benzène transformé.

Exemple 5

On opère comme à l'exemple 2, à 200° C pendant 1 h 20, si ce n'est que les quantités de chlorure cuivreux et d'hydroxy-8 quinoléine sont doublées, le solvant étant la tétraméthylurée.

Dans ces conditions, on obtient un taux de transformation de 76,4% et un rendement en dichloro-3,5 aniline de 50%, sur le trichloro-1,3,5 benzène transformé.

Exemple 6

On opère comme à l'exemple 1 en utilisant les trois mélanges suivants:

| | |
|---|---|
| Trichloro-1,3,5 benzène | 1,0 g |
| Ammoniaque à 28% | 2,9 g |
| Chlorure cuivreux | 0,03 g |
| Hydroxy-8 quinoléine (dans deux cas) | 0,085 g |
| Tétraméthylurée (dans un seul cas) | 2,0 g |

Le tableau suivant rassemble pour chaque mélange le taux de transformation du trichloro-1,3,5 benzène et le rendement en dichloro-3,5 aniline par rapport au trichloro-1,3,5 benzène transformé.

| Mélange | Hydroxy-8 quinoléine | Tétraméthyl-urée | TT % | RT % |
|---|---|---|---|---|
| 6A | — | — | 16,1 | 47 |
| 6B | ou | — | 27,0 | 40 |
| 6C | oui | oui | 61,9 | 60 |

Exemple 7

On opère comme à l'exemple 1, en utilisant le mélange de départ suivant:

| | |
|---|---|
| monochlorobenzène | 1,0 g ($8,9 \times 10^{-3}$ mole) |
| Ammoniaque à 28% | 2,9 g |
| Chlorure cuivreux | 0,03 g ($0,3 \times 10^{-4}$ mole) |
| Complexant | ($0,6 \times 10^{-4}$ mole) |
| Solvant | 2 g |

Le tableau suivant rassemble, pour chaque essai, la nature du complexant et du solvant ainsi que le taux de transformation du monochlorobenzène et le rendement en aniline par rapport au monochlorobenzène transformé:

| Complexant | Solvant | TT % | RT % |
|---|---|---|---|
| — | — | 14,6 | 80,9 |
| Hydroxy-8 quinoléine | éthanol | 23,5 | 72,2 |
| Acide hydroxy-8 quinoléine sulfonique-5 | éthanol | 30,6 | 80,4 |
| Hydroxy-8 quinoléine | tétraméthylurée | 28,7 | 80,0 |
| Acide hydroxy-8 quinoléine sulfonique-5 | tétraméthylurée | 36,9 | 87,0 |
| *) Acide hydroxy-8 quinoléine sulfonique-5 | tétraméthylurée | 57,6 | 91,5 |

*) Dans cet essai la durée de réaction a été de 2 h 50 minutes.

Ce tableau montre clairement que l'utilisation du complexant associé au solvant permet d'améliorer considérablement le taux de transformation tout en maintenant un rendement RT de haut niveau.

**Revendications**

1. Procédé de préparations d'anilines éventuellement halogénées, de formule générale:

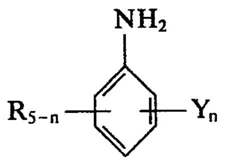

dans laquelle:

— Y représente un ou des atomes d'halogènes, identiques ou différents,
— R représente un ou plusieurs substituants choisis dans le groupe comprenant un atome d'hydrogène et un radical alcoyle contenant de 1 à 4 atomes de carbone ou alcoxyle contenant de 1 à 4 atomes de carbone et
— n est un nombre entier de 0 à 5, par ammonolyse d'halogénobenzènes de formule:

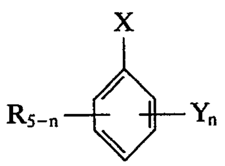

dans laquelle:

— Y et R ont les mêmes significations que précédemment et
— X représente un atome d'halogène, avec une solution aqueuse d'ammoniac, en présence de cuivre comme catalyseur et d'un complexant du cuivre, caractérisé en ce qu'on utilise comme complexant un dérivé de l'hydroxy-8 quinoléine.

2. Procédé selon la revendication 1 caractérisé en ce que le complexant est l'hydroxy-8 quinoléine.
3. Procédé selon la revendication 1 caractérisé en ce que le complexant est l'acide hydroxy-8 quinoléine sulfonique-5.
4. Procédé selon l'une des revendications 1 à 3, caractérisé en ce que le rapport molaire complexant/cuivre est compris entre 0,1/1 et 5/1.
5. Procédé selon la revendication 4, caractérisé en ce que le rapport molaire complexant/cuivre est compris entre 0,5/1 et 2/1.
6. Procédé selon l'une des revendications 1 à 5, caractérisé en ce que le milieu réactionnel contient en outre un solvant miscible à l'eau et sans effet désactivant du cuivre.
7. Procédé selon l'une des revendications 1 à 6, caractérisé en ce que le solvant est choisi dans le groupe comprenant la N-méthylpyrrolidone, la tétraméthylurée, la pyridine, un polyéthylène glycol de poids moléculaire de 400 à 1500 l'éthanol et le diglyme.
8. Procédé selon l'une des revendications 6 à 7, caractérisé en ce que le rapport pondéral solvant/halogénobenzène est compris entre 0,1/1 et 10/1.
9. Procédé selon la revendication 8, caractérisé en ce que le rapport pondéral solvant/halogénobenzène est compris entre 0,5/1 et 4/1.
10. Procédé selon l'une des revendications 1 à 9 caractérisée en ce que, dans la formule, X est un atome de chlore.
11. Procédé selon l'une des revendications 1 à 9, caractérisé en ce que, dans la formule, Y est un atome de chlore.
12. Procédé selon l'une des revendications 1 à 9, caractérisé en ce que, dans les formules, R est un atome d'hydrogène.
13. Procédé selon l'une des revendications 1 à 9, caractérisé en ce que n est un entier de 0 à 3.
14. Procédé selon l'une des revendications 10 à 13, caractérisé en ce que la formule X et Y sont un atome de chlore et n un entier de 0 à 3.

**Patentansprüche**

1. Verfahren zur Herstellung gegebenenfalls halogenierter Aniline der allgemeinen Formel

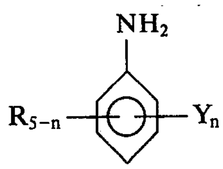

in der bedeuten:

- Y ein oder mehrere gleiche oder verschiedene Halogenatome,
- R einen oder mehrere unter Wasserstoff, $C_1$- bis $C_4$-Alkylgruppen und $C_1$- bis $C_4$-Alkoxygruppen ausgewählte Substituenten und
- n eine ganze Zahl von 0 bis 5

durch Ammonolyse von Halogenbenzolen der Formel

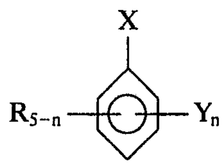

in der bedeuten:

- Y, R und n dasselbe wie oben und
- X ein Halogenatom

mit einer wäßrigen Ammoniaklösung in Gegenwart von Kupfer als Katalysator sowie eines Komplexbildners für Kupfer,
dadurch gekennzeichnet, daß als Komplexbildner ein Derivat des 8-Hydroxychinolins verwendet wird.

2. Verfahren nach Anspruch 1, dadurch gekennzeichnet, daß als Komplexbildner 8-Hydroxychinolin verwendet wird.
3. Verfahren nach Anspruch 1, dadurch gekennzeichnet, daß als Komplexbildner 8-Hydroxychinolin-5-sulfonsäure verwendet wird.
4. Verfahren nach einem der Ansprüche 1 bis 3, dadurch gekennzeichnet, daß ein Molverhältnis von Komplexbildner zu Kupfer von 0,1 : 1 bis 5 : 1 angewandt wird.
5. Verfahren nach Anspruch 4, dadurch gekennzeichnet, daß ein Molverhältnis von Komplexbildner zu Kupfer von 0,5 : 1 bis 2 : 1 angewandt wird.
6. Verfahren nach einem der Ansprüche 1 bis 5, dadurch gekennzeichnet, daß das Reaktionsmedium ferner ein mit Wasser mischbares Lösungsmittel ohne Desaktivierungswirkung gegenüber Kupfer enthält.
7. Verfahren nach einem der Ansprüche 1 bis 6, dadurch gekennzeichnet, daß ein unter n-Methylpyrrolidon, Tetramethylharnstoff, Pyridin, Polyethylenglycolen mit Molekulargewichten von 400 bis 1500, Ethanol und Diglym ausgewähltes Lösungsmittel verwendet wird.
8. Verfahren nach einem der Ansprüche 6 und 7, dadurch gekennzeichnet, daß ein Gewichtsverhältnis von Lösungsmittel zu Halogenbenzol von 0,1 : 1 bis 10 : 1 angewandt wird.
9. Verfahren nach Anspruch 8, dadurch gekennzeichnet, daß ein Gewichtsverhältnis von Lösungsmittel zu Halogenbenzol von 0,5 : 1 bis 4 : 1 angewandt wird.
10. Verfahren nach einem der Ansprüche 1 bis 9, dadurch gekennzeichnet, daß X in den Formeln ein Chloratom bedeutet.
11. Verfahren nach einem der Ansprüche 1 bis 9, dadurch gekennzeichnet, daß Y in den Formeln ein Chloratom bedeutet.
12. Verfahren nach einem der Ansprüche 1 bis 9, dadurch gekennzeichnet, daß R in den Formeln ein Wasserstoffatom bedeutet.
13. Verfahren nach einem der Ansprüche 1 bis 9, dadurch gekennzeichnet, daß n in den Formeln eine ganze Zahl von 0 bis 3 bedeutet.
14. Verfahren nach einem der Ansprüche 10 bis 13, dadurch gekennzeichnet, daß n in den Formeln X und Y jeweils ein Chloratom und n eine ganze Zahl von 0 bis 3 bedeuten.

## Claims

1. A process for the preparation of an optionally halogenated aniline of the general formula

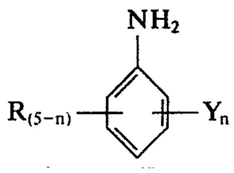

wherein:

- Y represents one or more identical or different halogen atoms,
- R represents one or more substituents selected from the group consisting of a hydrogen atom, an alkyl radical containing from 1 to 4 carbon atoms or an alkoxy radical containing from 1 to 4 carbon atoms, and
- n is an integer from 0 to 5,
  which comprises the ammonolysis of a halogenobenzene of the general formula:

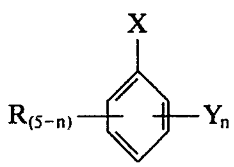

wherein

- Y, R and n have the same meanings as above and X represents a halogen atom,

with an aqueous solution of ammonia, in the presence of copper as a catalyst and of a complexing agent for the copper, characterised in that an 8-hydroxyquinoline derivative as the complexing agent is used.

2. A process according to claim 1 wherein the complexing agent is 8-hydroxyquinoline.

3. A process according to claim 1 wherein the complexing agent is 8-hydroxyquinoline-5-sulphonic acid.

4. A process according to any one of claims 1 to 3 wherein the molar ratio of complexing agent/copper is between 0.1/1 and 5/1.

5. The process according to claim 4 wherein the molar ratio of complexing agent/copper is between 0.5/1 and 2/1.

6. A process according to any one of claims 1 to 5 wherein the reaction medium also contains a solvent which is water-miscible and has no deactivating effect on the copper.

7. A process according to any one of claims 1 to 6 wherein the solvent is selected from the group comprising N-methyl-pyrrolidone, tetramethylurea, pyridine, a polyethylene glycol with a molecular weight of 400 to 1500, ethanol and diglyme.

8. A process according to claims 6 or 7 wherein the weight ratio of solvent/halogenobenzene is between 0.1/1 and 10/1.

9. A process according to claim 8 wherein the weight ratio of solvent/halogenobenzene is between 0.5/1 and 4/1.

10. A process according to any one of claims 1 to 9 wherein X in the formula is a chlorine atom.

11. A process according to any one of claims 1 to 9 wherein Y in the formula is a chlorine atom.

12. A process according to any one of claims 1 to 9 wherein R in the formula is a hydrogen atom.

13. A process according to any one of claims 1 to 9 wherein n is an integer from 0 to 3.

14. A process according to any one of claims 10 to 13 wherein X and Y are chlorine atoms and n is an integer from 0 to 3.